(19) Europäisches Patentamt
European Patent Office
Office européen des brevets

(11) EP 1 162 265 A2

(12) **EUROPÄISCHE PATENTANMELDUNG**

(43) Veröffentlichungstag:
**12.12.2001 Patentblatt 2001/50**

(21) Anmeldenummer: **01250175.5**

(22) Anmeldetag: **17.05.2001**

(51) Int Cl.[7]: **C12N 15/12**, C12N 15/11, C07K 14/47, G01N 33/68, C12Q 1/68, A61K 38/00, A61K 48/00

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **31.05.2000 DE 10027170**

(71) Anmelder: **Schering Aktiengesellschaft**
**13353 Berlin (DE)**

(72) Erfinder:
- **Haendler, Bernard, Dr.**
  **13465 Berlin (DE)**
- **Weiss, Bertram, Dr.**
  **14163 Berlin (DE)**
- **Geserick, Christoph**
  **12163 Berlin (DE)**

(54) **Humanes PEM als Target für die Fertilitätskontrolle und die Alzheimertherapie**

(57) Die Erfindung betrifft das humane PEM-Polypeptid, das für die Spermienreifung und für die Alzheimer Krankheit eine wesentliche Rolle spielt und die dafür kodierende Nukleinsäure. Die Erfindung umfasst die Verwendung von PEM als Target in der männlichen Fertilitätskontrolle und für die Behandlung und Diagnose der männlichen Infertilität und der Alzheimer Krankheit. Zur Erfindung gehört auch ein Selektionsverfahren für PEM-Antagonisten sowie die Herstellung von Bindungsmolekülen, die spezifisch PEM erkennen. Weiterhin sind Gene, die durch das PEM-Gen reguliert sind, Teil dieser Erfindung.

EP 1 162 265 A2

## Beschreibung

Die Erfindung betrifft das humane PEM-Polypeptid, das für die Spermienreifung eine wesentliche Rolle spielt und die dafür kodierende Nukleinsäure. Die Erfindung umfasst die Verwendung von PEM als Target in der männlichen Fertilitätskontrolle und für die Behandlung und Diagnose der männlichen Infertilität und der Alzheimer-Krankheit. Zur Erfindung gehört auch ein Selektionsverfahren für PEM-Antagonisten sowie die Herstellung von Bindungsmolekülen, die spezifisch PEM erkennen. Weiterhin sind Gene, die durch das PEM-Gen reguliert sind, Teil dieser Erfindung.

Die Absicht, Proteine des männlichen Reproduktionstraktes oder Spermienproteine als Zielgruppe zur nicht-hormonellen Kontrazeption zu nutzen, ist seit mehreren Jahrzehnten bekannt. Zum Beispiel wurde von der Weltgesundheitsorganisation (WHO) ein Projekt mit dem Namen "Vaccines for fertility regulation" unterstützt (P.D. Griffin, Hum. Reprod., 1991, 6:166-172). Verschiedene Spermien-Proteine wie z. B. PH-20, SP-10, FA-1, FA-2, CS-1, NZ-1, NZ-2 und die Lactat-Dehydrogenase C4 wurden als Kandidaten für Immunokontrazeption vorgeschlagen (R. K. Naz, Immunol. Rev., 1999, 171:193-202). Immunisierungsversuche mit PH-20 zeigten, dass sowohl männliche als auch weibliche Tiere dadurch vollständig und reversibel infertil werden (P. Primakoff et al., Nature, 1988, 335:543-546). Der Einsatz des intraakrosomalen Spermienproteins SP-10 als Antigen ruft eine Immunantwort bei Frauen, die die Fertilität senkt, hervor (R.W. Wright et al., Biol. Reprod., 1990, 42:693-701). Aktive Immunisierung von Tieren mit FA-1 bewirkt eine andauernde und reversible Hemmung der Fertilität (R. K. Naz and X. Zhu, Biol. Reprod., 1998, 59:1095-1100).

PEM ist ein Transkriptionsfaktor, der zur Homeobox-Familie gehört. Die entsprechende cDNA wurde aus der Maus (M. F. Wilkinson et al., Dev. Biol., 1990, 141:451-455) und aus der Ratte (S. Maiti et al., J. Biol. Chem., 1996, 271:17536-17546) kloniert. PEM-Transkripte sind abundant und selektiv im männlichen Genitaltrakt exprimiert. In der Maus wurde die PEM-Expression hauptsächlich im Hoden nachgewiesen, während in der Ratte PEM hauptsächlich im Nebenhoden zu finden ist (K. A. Sutton et al., J. Androl., 1998, 19:21-30). Die in vivo Expression des PEM-Gens ist in diesen Organen durch Androgene reguliert. Weiterhin wurden PEM-Transkripte im Muskel und in Makrophagen beschrieben, aber in diesen Fällen scheint die PEM-Expression nicht durch Androgene reguliert zu sein, was sich auf die Benützung unterschiedlicher Promotoren zurückführen lässt (S. Maiti et al., J. Biol. Chem., 1996, 271: 17536-17546). Trotz des unauffälligen Phenotyps der PEM-Knock-Out-Maus (J. L. Pitman et al., Dev. Biol., 1998, 202: 196-214) ist es zu vermuten, dass das humane PEM eine essentielle Rolle in der Spermatogenese oder/und in der Spermienreifung spielt. PEM ist der einzig bekannte Transkriptionsfaktor, dessen Expression durch Androgene reguliert wird (S. Maiti et al., J. Biol. Chem., 1996, 271:17536-17546).

Bislang konnte das humane PEM-Ortholog nicht gefunden werden, was auf eine geringe Sequenzkonservierung in unterschiedlichen Organismen hinweist, wie bereits durch die schwache Identität (73 %) zwischen Maus-PEM und Ratten-PEM festzustellen ist (S. Maiti et al., Genomics, 1996, 34:304-316).

Die Erfindung betrifft die Identifizierung des humanen PEM. Es konnte sowohl die komplette kodierende PEM-cDNA-Sequenz wie auch die Struktur des PEM-Gens ermittelt werden. Die humane PEM-Aminosäuresequenz hat nur 30 % Identität mit der Sequenz aus Maus und nur 32 % Identität mit der Sequenz aus Ratte. Der humane genomische Lokus konnte zu Xq 25-26 definiert werden.

Die identifizierte cDNA-Sequenz ist in SEQ ID No. 1 und die Proteinkodierende Sequenz ist in SEQ ID No. 2 dargestellt. Die genomische Sequenz konnte ebenfalls identifiziert werden und ist in SEQ ID No. 3 dargestellt (entsprechend einem Ausschnitt der Nukleotide 16000- 170967 aus Genbank Accession No. AC005023). Das initiale Exon reicht von Nukleotid 168 439 bis 168 042. Ein internes Exon reicht von Nukleotid 165 491 bis 165 446 und das terminale Exon reicht von Nukleotid 161 927 bis 161 817 (111 Nukleotide). Im Bereich der Nukleotide 161 698 bis 161 693 befindet sich ein Polyadenylierungssignal.

Vorzugsweise wird das humane PEM von (a) dem kodierenden Bereich der in SEQ ID No. 1 gezeigten Nukleinsäuresequenz, (b) einer der Sequenz gemäß (a) im Rahmen der Degeneration des genetischen Codes oder/und (c) einer mit den Sequenzen gemäß (a) oder/und (b) unter stringenten Bedingungen hybridisierenden Nukleinsäuresequenz kodiert. Besonders bevorzugt besitzt das humane PEM die in SEQ ID No 2 gezeigte Aminosäuresequenz oder eine dazu mindestens 80 %, vorzugsweise mindestens 90 %, identische Aminosäuresequenz.

Der Begriff "stringente Hybridisierung" gemäß vorliegender Erfindung wird dabei wie bei Sambrook et al. (Molecular Cloning, A Laboratory Manual, Cold Spring Harbor, Laboratory Press (1989), 1.101-1.104) verwendet. Demnach spricht man von Hybridisierung unter stringenten Bedingungen, wenn nach Waschen für eine Stunde mit 1 x SSC und 0,1 % SDS bei 55°C, vorzugsweise bei 62°C und besonders bevorzugt bei 68°C, insbesondere für eine Stunde mit 0,2 X SSC und 0,1 % SDS bei 55°C, vorzugsweise bei 62°C und besonders bevorzugt bei 68°C, noch ein positives Hybridisierungssignal beobachtet wird. Eine unter derartigen Waschbedingungen mit einer in SEQ ID No. 1 gezeigten Nukleotidsequenz oder einer damit im Rahmen der Degeneration des genetischen Codes entsprechenden Nukleotidsequenz hybridisierende Sequenz wird von der vorliegenden Erfindung erfasst.

Insbesondere erfasst die vorliegende Erfindung natürliche, allelische Variationen von PEM, bei denen es sich gegebenenfalls auch um funktionelle Mutationen handeln kann. Darüber hinaus werden auch rekombinante Varianten, beispielsweise funktionelle Teilfragmente (wie etwa die "Divergent Paired Class" Homeodomäne wie für die Maus von Rayle (Develop. Biol. 146 (1991), 255-257) beschrieben) von der vorliegenden Erfindung erfasst.

Besonders bevorzugt weist das humane PEM die in SEQ ID No. 2 gezeigten Aminosäuresequenz oder eine dazu mindestens 80 % und insbesondere mindestens 90 % identische Sequenz auf. Die Identität I % wird dabei nach folgender Formel berechnet:

$$I = n/L \times 100\ \%,$$

wobei n für die Anzahl identischer Aminosäuren der beiden, miteinander verglichenen Sequenzen und L für die Länge des zum Vergleich herangezogenen Sequenzabschnitts steht.

Die Hemmung von PEM kann zur Hemmung der Spermien-Entstehung bzw. - Reifung führen und stellt somit einen neuartigen Weg für Kontrazeptivansätze dar. Weiterhin kann das Screening nach funktionellen Mutationen im PEM-Gen als diagnostisches Mittel zur Bestimmung der Ursachen von Infertilität verwendet werden. Durch Wiederherstellen der PEM-Funktion (z.B. durch Gentherapie) kann die Fertilität der Patienten wieder hergestellt werden.

Ein Gegenstand der Erfindung ist somit die Verwendung von humanem PEM oder einer dafür kodierenden Nukleinsäure als Zielsubstanz zur Herstellung eines Mittels für die Fertilitätskontrolle.

Eine Hemmung von humanem PEM kann zur Hemmung der Fertilität und insbesondere zur Hemmung der Spermatogenese bei männlichen Säugern eingesetzt werden. Dies ist von großer Bedeutung bei der humanen Empfängnisverhütung, aber auch in der Veterinärmedizin zur Populationskontrolle. Die Hemmung von PEM kann durch Expressionsverringerung mittels Antisense-Nukleinsäuren oder Ribozymen oder auf Proteinebene durch Verwendung von Inhibitoren wie Anti-PEM-Antikörpem oder niedermolekularen Antagonisten erfolgen. Die Herstellung von Antisense-Molekülen und Ribozymen kann beispielsweise wie bei Sczakiel (Antisense Nucleic Acid Drug Dev. 7 (1997), 439-444, Lavrovsky et al. (Biochem. Mol. Med. 62 (1997), 11-22) und Thompson (Methods Enzymol. 306 (1999), 241-260) beschrieben erfolgen. Polyklonale Antikörper gegen humanes PEM können durch Immunisierung von Versuchstieren mit humanem PEM oder Fragmenten davon, gegebenenfalls an einen Träger wie Keyhole-Limpet-Hämocyanin, und Gewinnung der resultierenden Antikörper aus dem immunisierten Versuchstier erfolgen. Monoklonale Antikörper können beispielsweise durch Fusion von Milzzellen des immunisierten Versuchstiers mit Myelomzellen nach der Methode von Köhler und Milstein oder Weiterentwicklungen davon erhalten werden. Niedermolekulare Inhibitoren von PEM können durch ein Screening Verfahren wie im Folgenden ausführlich erläutert, identifiziert werden.

Andererseits kann eine Aktivierung von humanem PEM zur Erhöhung der Fertilität eingesetzt werden. Auch hier sind Anwendungen sowohl in der Humanmedizin als auch in der Veterinärmedizin möglich. Die Aktivierung von PEM kann beispielsweise durch Erhöhung der PEM-Expression in Zielzellen, z.B. Sertoli-Zellen im Hoden oder/und Epithelzellen im Nebenhoden, mittels gentherapeutischer Methoden erfolgen. Hierzu kann eine für PEM kodierende Nukleinsäure unter Kontrolle eines in der Zielzelle aktiven Promotors mittels geeigneter Gentransfervektoren, z.B. viraler Vektoren wie etwa Adenoviren, Retroviren, Adeno-assoziierten Viren oder Vacciniaviren, oder Plasmiden, in die Zielzelle eingeführt und dort zur Expression gebracht werden. Geeignete gentherapeutische Verfahren sind z.B. in Gomez-Navarro et al. (Eur. J. Cancer, 35 (1999), 867-885) beschrieben. Weiterhin kann eine Aktivierung von PEM durch niedermolekulare Wirksubstanzen erfolgen, die durch ein wie im Folgenden beschriebenes Screening Verfahren identifiziert werden können.

Ein weiterer Gegenstand der Erfindung ist ein Verfahren zur Bereitstellung von neuen Mitteln für die Fertilitätskontrolle. Die Identifizierung dieser neuen Mittel erfolgt dadurch, dass man die Fähigkeit von Testsubstanzen zur Modulation von humanem PEM bestimmt. Diese Bestimmung kann als Hochdurchsatz-Test durchgeführt werden, bei dem eine Vielzahl von Testsubstanzen parallel untersucht wird. Der Test kann auf zellulärer Basis durchgeführt werden, wobei Zellen verwendet werden können, die mit dem Gen für das humane PEM transfiziert sind und in der Lage sind, eine Überexpression dieses Gens zu bewirken. Andererseits können auch Zellen getestet werden, die ein vollständig oder teilweise defektes PEM enthalten, beispielsweise Zellen, die in mindestens einem Allel, vorzugsweise in beiden Allelen ein defektes, humanes PEM Gen enthalten. Die für die Identifizierung neuer Wirksubstanzen verwendeten Testzellen sind vorzugsweise Säugerzellen, insbesondere humane Zellen. Alternativ kann ein Test auf molekularer Basis durchgeführt werden, wobei das humane PEM in Form von Zellextrakten oder in einer im Wesentlichen isolierten und gereinigten Form, gegebenenfalls auch in Form eines aktiven Fragments, eingesetzt wird.

Das erfindungsgemäße Verfahren zur Identifizierung von neuen Mitteln zur Fertilitätskontrolle kann weiterhin die Formulierung von auf humanes PEM modulatorisch wirkenden Testsubstanzen oder davon abgeleitete Verbindungen zu einem Arzneimittel umfassen.

Noch ein weiterer Gegenstand der Erfindung ist ein Diagnostikverfahren, bei dem die Expression oder/und die Funktionalität von humanem PEM in einer Probe bestimmt wird. Die Probe stammt vorzugsweise von einem Patienten, der einer Fertilitätsbestimmung unterzogen werden soll oder von einem Patienten, bei dem der Verdacht auf eine Alzheimer Krankheit vorliegt.. Die Bestimmung von PEM kann auf Nukleinsäureebene, z.B. auf DNA-Ebene beispielsweise durch Southem Blot oder Bestimmung von Einzelnukleotid-Polymorphismen, auf Transkriptebene durch Bestimmung der Expressionshöhe, des Expressionsmusters oder der Transkriptlänge oder auf Proteinebene, z.B. durch immunhistochemische oder immuncytochemische Methoden oder durch Funktionsmessungen erfolgen. Die Bestimmung von Einzelnukleotid-Polymorphismen erlaubt die Identifizierung und Diagnostik funktioneller Mutationen, welche in Patienten ursächlich für eine Infertilität sein können.

Zusätzlich zu der Rolle in der Spermienreifung hat das humane PEM auch eine Funktion bei der Alzheimer Krankheit. Humanes PEM wird im Gehirn von Alzheimer-Patienten erhöht exprimiert im Vergleich zu Gehirnen von gesunden Menschen. Die Hemmung von PEM kann sich positiv auf den Verlauf der Alzheimer-Krankheit auswirken und stellt somit einen neuen Ansatz für die Behandlung dieser Krankheit dar. Ein Gegenstand der Erfindung ist daher die Verwendung von humanen PEM oder einer dafür kodierenden Nukleinsäure als Zielsubstanz zur Herstellung eines Mittels zur Behandlung der Alzheimer-Krankheit. Die Hemmung von PEM kann durch eine Verminderung der Expression von PEM erfolgen. Dies kann durch Antisense-Nukleinsäuren, Ribozyme oder durch Substanzen, die in den Regulationsmechanismus der PEM-Genexpression eingreifen, erfolgen. Solche Substanzen lassen sich durch ein Testsystem, das die PEM-Genexpression mißt, identifizieren. So können z.B. Zellen, die mit der PEM-DNA transfiziert sind mit den zu testenden Substanzen in Kontakt gebracht werden und die Expression des PEM-Proteins z.B. mit Hilfe von Antikörpern nachgewiesen werden.

Weiterhin kann PEM auch auf Proteinebene gehemmt werden, z.B. durch Antikörper, Peptide oder niedermolekulare Antagonisten. Da PEM ein Transkriptionsfaktor ist, ist es möglich, die Bindung von PEM an DNA oder die Interaktion mit der Transkriptionsmaschinerie zu hemmen.

Gegenstand der Erfindung ist auch ein Verfahren zur Bereitstellung von neuen Mitteln für die Behandlung der Alzheimer-Krankheit. Die Identifizierung dieser neuen Mittel erfolgt dadurch, dass die Fähigkeit von Testsubstanzen zur Hemmung von humanen PEM bestimmt wird. Gemessen wird die Funktion des PEM als Transkriptionsfaktor. Es kann die Bindung von PEM an DNA gemessen werden. Andererseits können auch Zellen verwendet werden, die mit dem Gen für das humane PEM transfiziert sind. In diesen Zellen ist das PEM-Protein für die Genregulation anderer Gene, sogenannter Zielgene, verantwortlich. Eine Hemmung, von PEM durch die Testsubstanzen führt zu einer Verminderung der Expression der Zielgene.

Ein weiterer Gegenstand der Erfindung ist eine Zelle, die mit einer für das humane PEM kodierenden DNA oder einem Fragment davon transfiziert ist und mindestens eine exogene Kopie dieser DNA enthält. Noch ein weiterer Gegenstand der Erfindung ist eine Zelle, die in mindestens einem Allel ein defektes PEM-Gen enthält, beispielsweise ein mittels homologer Rekombination disruptiertes PEM-Gen. Diese Zellen können ebenso wie die Nukleinsäuren, die für humanes PEM oder ein Fragment davon kodieren, oder das humane PEM-Protein selbst oder ein Fragment davon zur Identifizierung und Charakterisierung von Mitteln zur Fertilitätskontrolle und zur Behandlung der Alzheimer-Krankheit eingesetzt werden.

Schließlich betrifft die Erfindung ein Verfahren zur Identifizierung von Genen, die durch das humane PEM-Gen reguliert sind, wobei man den Einfluss von humanem PEM auf die Genexpression in humanen Zellen testet. Dieses Testen kann beispielsweise durch Transkriptomanalyse, z.B. nach den von Kozian und Kirschbaum (Trends Biotechnol. 17 (1999), 73-78) beschriebenen Methoden oder durch Proteomalyse nach den von Dutt und Lee (Curr. Opin. Biotechnol. 11 (2000), 176-179) beschriebenen Methoden erfolgen. Die durch das Verfahren identifizierten Gene und deren Verwendung als Zielsubstanz zur Herstellung eines Mittels für die Fertilitätskontrolle oder zur Behandlung der Alzheimer-Krankheit sind ebenfalls Gegenstand der vorliegenden Erfindung.

Die Erfindung wird durch die nachfolgende Abbildungen und Beispiele näher erläutert.

**Beschreibung der Abbildung**

Fig. 1 zeigt die Expression von humaner PEM mRNA in Gewebeproben aus verschiedenen Teilen des Gehirns.

A. Ein sense Primer sowie ein antisense Primer gegen den proteinkodierenden Abschnitt der humanen PEM-RNA wurden für die PCR-Amplifizierung verwendet. Als Template diente first-strand cDNA aus verschiedenen Gehirnteile. Die PCR-Produkte wurden auf einem 1,5 % Agarosegel getrennt und anschließend mit Ethidiumbromid gefärbt. Das humane PEM Amplifikat ist bei einer Größe von ca. 550 bp zu erkennen. Als Größenmarker wurden der 1 kb Marker sowie der 100 bp Marker von Clontech aufgetragen. 1: 100 bp Marker; 2: gesundes Gehirn, Probe-a; 3: gesunder temporal lobe; 4: Alzheimer erkrankter temporal lobe; 5: gesunder frontal lobe; 6: Alzheimer erkrankter frontal lobe; 7: gesunder Hypocampus; 8: tumor Hypocampus; 9: fötales Gehirn; 10: gesundes Gehirn, Probe-b; 11: Wasserkontrolle; 12: 100 bp Marker; 13: 1 kb Marker.

B. Ein sense Primer sowie ein antisense Primer gegen den proteinkodierenden Abschnitt der Beta-Aktin-RNA wurden für die PCR-Amplifizierung verwendet. Als Template diente die gleiche first-strand cDNA wie unter A. 1: 100-bp-Marker; 2: 1-Kb Marker; 3: gesundes Gehirn, Probe-a; 4: gesunder Hypocampus; 5: tumor Hypocampus; 6:: gesunder temporal lobe; 7: gesunder frontal lobe; 8: Alzheimer erkrankter temporal lobe; 9: Alzheimer erkrankter frontal lobe; 10: fötales Gehirn; 11: gesundes Gehirn, Probe-b; 12: Wasserkontrolle.

**Beispiel**

Die Expression des humanen PEM in diversen Gehirngeweben wurde durch semiquantitative PCR analysiert. Hierzu wurden folgende Primer verwendet: Sense 5'-ATGGCGCGTTCGCTCGTCCACGAC-3',
Antisense 5'-TAGTCCACGACGATGTAGACACAG-3'.
In der Kontrolle wurden spezifische Primer für Beta-Actin verwendet: Sense Primer 5'-CTAGAAGCATTTGCGGTGGACGATGGAGGG-3', Antisense Primer 5'-CTAGAAGCATTTGCGGTGGACGATGGAGGG-3'. Die cDNA wurde von Invitrogen (Carlsbad, CA, USA) erworben. Die PCR Analyse wurde mit dem Advantage-2 PCR kit (Clontech) durchgeführt. Die Reaktionsbedingungen waren wie folgt: Initial 5 min 95°C; anschließend 30 Zyklen mit 95°C für 30 sec, 62°C 30 sec sowie 72°C für 1 min; abschließend 72°C 7 min. Die Ergebnisse zeigen eine erhöhte Expression von humanem PEM im frontalen- sowie temporalen lobe bei Alzheimer Patienten im Vergleich zu gesunden Gewebe.

## Patentansprüche

**1.** Nukleinsäure mit einer (a) in Seq ID No 1 dargestellten Nukleotidsequenz, (b) einer der Sequenz aus (a) im Rahmen der Degeneration des genetischen Codes entsprechenden Nukleotidsequenz oder (c) einer mit den Sequenzen aus (a) oder (b) unter stringenten Bedingungen hybridisierende Nukleotidsequenz.

**2.** Polypeptid mit einer in Seq ID No 2 dargestellten Aminosäuresequenz.

**3.** Verwendung von humanem PEM oder einer dafür kodierenden Nukleinsäure als Zielsubstanz zur Herstellung eines Mittels für die Fertilitätskontrolle.

**4.** Verwendung von humanem PEM oder einer dafür kodierenden Nukleinsäure als Zielsubstanz zur Herstellung eines Mittel für die Behandlung der Alzheimer-Krankheit.

**5.** Verwendung nach Anspruch 3 oder 4, **dadurch gekennzeichnet, dass** das humane PEM von (a) dem kodierenden Bereich der in SEQ ID No. 1 gezeigten Nukleinsäuresequenz , (b) einer der Sequenz gemäß (a) im Rahmen der Degeneration des genetischen Codes oder/und (c) einer mit den Sequenzen gemäß (a) oder/und (b) unter stringenten Bedingungen hybridisierenden Nukleinsäuresquenz kodiert ist.

**6.** Verwendung nach Anspruch 3, 4 oder 5,
**dadurch gekennzeichnet,**
**dass** das humane PEM die in SEQ ID No. 2 gezeigte Aminosäuresequenz oder eine dazu mindestens 80 % identische Aminosäuresequenz aufweist.

**7.** Verwendung nach einem der Ansprüche 3, 5 oder 6,
**dadurch gekennzeichnet,**
**dass** eine Hemmung von PEM zur Verringerung der Fertilität eingesetzt wird.

**8.** Verwendung nach einem der Ansprüche 3, 5 oder 6,
**dadurch gekennzeichnet,**
**dass** eine Aktivierung von PEM zur Erhöhung der Fertilität eingesetzt wird.

**9.** Verfahren zur Identifizierung von Mitteln für die Fertilitätskontrolle,
**dadurch gekennzeichnet,**
**dass** man die Fähigkeit von Testsubstanzen zur Modulation von PEM bestimmt.

**10.** Verwendung nach Anspruch 4, 5 oder 6 **dadurch gekennzeichnet, dass** eine Hemmung von PEM zur Behandlung der Alzheimer-Krankheit eingesetzt wird.

**11.** Verfahren zur Identifizierung von Mitteln zur Behandlung der Alzheimer-Krankheit, **dadurch gekennzeichnet,**

**dass** die Fähigkeit von Testsubstanzen zur Hemmung von PEM bestimmt wird.

**12.** Verfahren nach einem der Ansprüche 9 oder 11,
weiterhin umfassend die Formulierung der modulatorisch oder inhibitorisch wirkenden Testsubstanzen oder davon abgeleitete Verbindungen zu einem Arzneimittel.

**13.** Verfahren zur Fertilitätsdiagnostik,
**dadurch gekennzeichnet,**
**dass** man die Expression oder/und Funktionalität von humanem PEM in einer Probe bestimmt.

**14.** Zelle,
**dadurch gekennzeichnet,**
**dass** sie mit einer für humanes PEM oder ein Fragment davon kodierenden Nukleinsäure transfiziert ist.

**15.** Humane Zelle,
**dadurch gekennzeichnet,**
**dass** sie in mindestens einem Allel ein defektes PEM-Gen enthält.

**16.** Verfahren zur Identifizierung von Genen, die durch das humane PEM-Gen reguliert sind,
**dadurch gekennzeichnet,**
**dass** man den Einfluss von humanen PEM auf die Genexpression in humanen Zellen testet.

**17.** Verfahren nach Anspruch 16,
**dadurch gekennzeichnet,**
**dass** man eine Transkriptoren- oder Proteomanalyse durchführt.

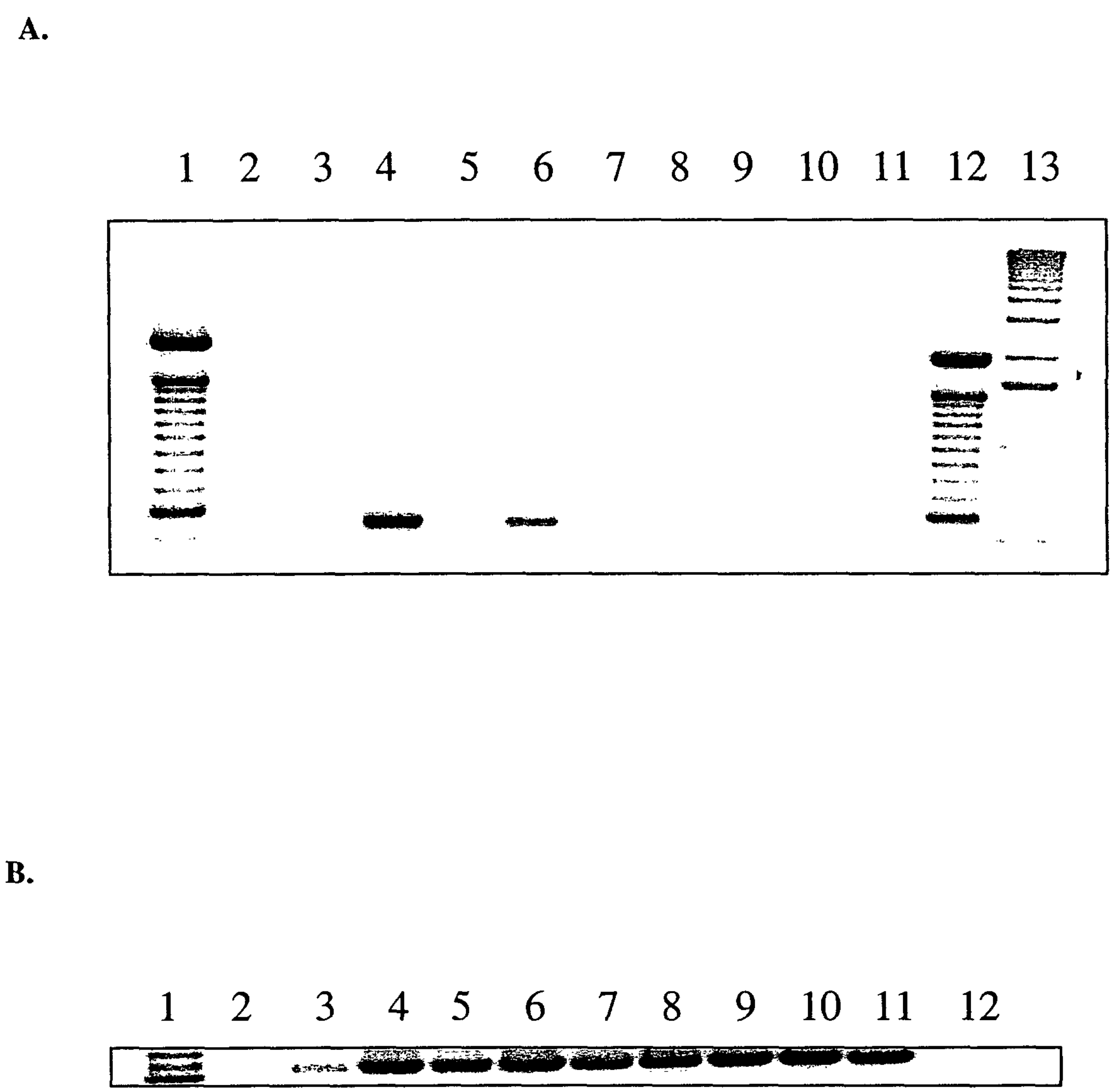


Fig. 1

SEQUENZPROTOKOLL

<110> Schering AG

<120> Humanes PEM als Target für die Fertilitätskontolle

<130> 51852

<140> 100 27 170.7
<141> 2000-05-31

<160> 3

<170> PatentIn Ver. 2.1

<210> 1
<211> 577
<212> DNA
<213> Homo sapiens

<400> 1
tccaacatca ggcgctccag ccatggcgcg ttcgctcgtc cacgacaccg tgttctactg 60
cctgagtgta taccaggtaa aaataagccc cacacctcag ctgggggcag catcaagcgc 120
agaaggccat gttggccaag gagctccagg cctcatgggt aatatgaacc ctgagggcgg 180
tgtgaaccac gagaacggca tgaaccgcga tggcggcatg atccccgagg gcggcggtgg 240
aaaccaggag cctcggcagc agccgcagcc cccgccggag gagccggccc aggcggccat 300
ggagggtccg cagcccgaga acatgcagcc acgaactcgg cgcacgaagt tcacgctgtt 360
gcaggtggag gagctggaaa gtgttttccg acacactcaa taccctgatg tgcccacaag 420
aagggaactt gccgaaaact taggtgtgac tgaagacaaa gtgcgggttt ggtttaagaa 480
taaaagggcc agatgtaggc gacatcagag agaattaatg ctcgccaatg aactacgtgc 540
tgacccagac gactgtgtct acatcgtcgt ggactag 577

<210> 2
<211> 184
<212> PRT
<213> Homo sapiens

<400> 2
Met Ala Arg Ser Leu Val His Asp Thr Val Phe Tyr Cys Leu Ser Val
1 5 10 15

Tyr Gln Val Lys Ile Ser Pro Thr Pro Gln Leu Gly Ala Ala Ser Ser
20 25 30

Ala Glu Gly His Val Gly Gln Gly Ala Pro Gly Leu Met Gly Asn Met
35 40 45

Asn Pro Glu Gly Gly Val Asn His Glu Asn Gly Met Asn Arg Asp Gly
50 55 60

Gly Met Ile Pro Glu Gly Gly Gly Gly Asn Gln Glu Pro Arg Gln Gln
65 70 75 80

Pro Gln Pro Pro Pro Glu Glu Pro Ala Gln Ala Ala Met Glu Gly Pro
85 90 95

Gln Pro Glu Asn Met Gln Pro Arg Thr Arg Arg Thr Lys Phe Thr Leu
100 105 110

Leu Gln Val Glu Glu Leu Glu Ser Val Phe Arg His Thr Gln Tyr Pro
115 120 125

```
Asp Val Pro Thr Arg Arg Glu Leu Ala Glu Asn Leu Gly Val Thr Glu
    130                 135                 140

Asp Lys Val Arg Val Trp Phe Lys Asn Lys Arg Ala Arg Cys Arg Arg
145                 150                 155                 160

His Gln Arg Glu Leu Met Leu Ala Asn Glu Leu Arg Ala Asp Pro Asp
                165                 170                 175

Asp Cys Val Tyr Ile Val Val Asp
            180


<210> 3
<211> 10968
<212> DNA
<213> Homo sapiens

<400> 3
caatacaaga gaatgtctgt gttaagataa ggggttgtgg agaccaaggt tcccattatg 60
cagaggaagc ctccaggtag ctggcttcag agagaataga ttgtaaatgt ttcttacttg 120
agttgattct ctcctggatc aagaaaaagg cctgcacaag aaaggggatt ctcttgagaa 180
tgtacatttc cccccacaag agacagcttt gcaggactgt ttcaaaatat gacaaagaaa 240
cacatagggt aaaatacttt tgatttcttt caagccttgc tatctgtcat gtgatgctat 300
actagagtta ggctggaaat tggtgtctta ttgccacaga gtatgttagt cttaagttct 360
gttctaacgt taagactggt cagctgtaca cgaattccaa aagggagtag ggaataataa 420
ggcatgtctg acgcctactt cctgtcatga cctgaataag tttttcaggt taactttgga 480
atgcccttgg ctgagaggag ggatccattc agatagttgt ggggcttcga attttatttt 540
tggtttacaa tagcatgaac aaagcagagg tctgacagct tcgttccagt gagtggatat 600
tctggaacat tgctcagggt accatcttct tactcttctt tgagcagcac taaatgaaaa 660
ggtccccttt caccttgtaa tcagcaggaa gtgggattct ctcgaagatg ttgaagatga 720
caaaataaac ttaaaggatt gttcatctgc ttttgagcta gggaaggtat aacaatatgc 780
tttctgggcc ggggggaggg gagaaaatgg agaagagcct ctttttgggc ttaatgaaat 840
ttttgcttgt gtttcttttg aagcagcagg atctttgggg cagaatagct cctattcccc 900
tgtgtccccc acaaaaaggg agggcagtga acagaatttg gagcatagtg gagtggatca 960
acgttcagct gccaccttcc cataaatcct atgagtagcc acctaggaag tttctcttta 1020
gagtccagaa tttggactga actagtcagc ataactggaa ctcagcttta tctgggaata 1080
cactgttgtc tcaccaggaa tctgcttcac cccttcttgc acatatttgt ggtccctaaa 1140
ggggcaaggt ggtgaggatg gcataatggc aggggtaggg agggggagtg gagaaggatg 1200
tatgggtcag tgcaaactca caatgacgct tggtaaactt ctgtgatgtg cagggcctat 1260
tgttgatggc aagccaggga tgtcatttca tgaaagatct ccttgtcatt ttgtttaaat 1320
ggctttcttt tttttttttt ttgatatgga gtctcactct gttgcccagg ctgaagtgca 1380
gtggtgcgat cttggctcac tgcaacctct gcctcctggg ttcaggcctc ccgcatagct 1440
gggattactg gtgcctgcca ccacatccag ctaatttttt tgtatttttg atagagacag 1500
ggtttcacca tcttggctag gctggtcttg aactcctgac ctcctgatcc acccgcctca 1560
gcctcctaaa gtgttaagat tacaggtgtg agccactgca cctggcctta aatggctttt 1620
taaaaacaat ttgcacctat accctactaa ccacaattgg cacacaaaaa caaatatatt 1680
gagaatttgc ctctttattg ataacataag tgcagaggag ataagggtag cctgagcggc 1740
atgggcagcc caggtgtcag tggcaccaga aaaacccatc tccaaactag ctcctgaaga 1800
aggatggcat tctagggcta gtccacgacg atgtagacac agtcgtctgg gtcagcacgt 1860
agttcattgg cgagcattaa ttctctctga tgtcgcctac atctggccct tttattctta 1920
aaccaaacct acaatcagag ggaaaagggg attggtttag tatattgaac agttaatgtc 1980
gtaatagaaa aacacaggat gcaactttat atgctattga gattttaaac tgcatcagga 2040
aaagctattt cctcattgct aaaatacctt aggaaagtta acaacatagc ccgtggccct 2100
tcagctcacc cttagtgagg accagctttg tgccaagtcc tggaataagc ttattacttt 2160
gtatctctct tctccatttt atttatttat ttatttatta tttatttatt tatttattta 2220
tttatttttt gagacagggt cttgctgttt tgcctgggct gggatccagt ggtgcaatca 2280
tagctcactg tgacattgaa cttctgggct caagagatcc tcccacctca ccctcccaag 2340
tagctggtac tagaggtaca tgccactatg cccagctgtt ttaatttttc tgtagagaca 2400
gggtctcgct atgttgccca ggctggactt gagctcctgg cctcaagtga tcttcccacc 2460
ttggtgtccc aaagtgttgg gattacaagc gtgagccact gtgcccagcc ccaattttaa 2520
tattctttaa tggttacttc cagatattgg atgcagttct ggcttatgag ttgttccagg 2580
tccttgctgt ttgttaattc aatgcctggc aacagggtaa caaaaggtgt gcatctgaca 2640
```

agtgaccatc aactatccag ctgcctcctg ctccctcctc actagggaga gtttcatctt 2700
gtttgtggga gaagttcggc atggtaaaaa gtgggcctaa tttcaaatca ttttcagggg 2760
attgtttaaa aaatccatct ttagtatgta gtaaataata ggaaagagcg cactggaatt 2820
ttagacaggt ttccttccag gatgtctaag ggatcattcg tcctctggca agagaggcct 2880
ggacactgcc ttgatatttt agcctgtagc attaaggaaa gttgaaacca gctcgaccca 2940
aattaactga aactctcaaa aatctttgct cacccaatag tttaggggaa agaggcatac 3000
cattgtcacc aatgccaaat cttcgttctc caatctgctg cactctccaa accttcctgg 3060
gctcaggaca aggtcagctc actctgtttt acctacagct ccaggatcct ggactggagg 3120
tgctgtagcc cagtaaggca gggcccccta ggccctgcta ctcaaccagg agatctgaat 3180
cccacccccct attcctaagg cagaaaggtg gaaccagcat tttaggaaga tggttaacat 3240
caatgtgggg gaagggtcac aaatatggct cctccctaaa tatctgccaa caattaaaaa 3300
gcaaacagac aaaaaaagcc tgtcagttag atgtcactat cctctcagca acctagttaa 3360
cggagtttat attgtattta ttactttcaa aagttctcaa actgcaaatt gtaagctgca 3420
caaagggcct tctttctcta cctgacacgt ctttttcact ttcccagtta aggatttgca 3480
gtatttctgc tgcatgaggc cagtctctaa aagtctaaaa gagctcattt tgggagcttt 3540
caagtgtacc actggtcaaa tctctataaa cataaccaaa gtgtacagtg ggttaactgg 3600
tatgttctga tactaggtct gcattcccaa tactggtttc ataaaccagt tgcattacat 3660
ctgcaaaagc tatggggaaa ctatgtatta ctttcttggg ggaaatttat gctgtatagt 3720
ttggagatac atgagagcat tctgtctctt cccttatttg tatcttgtgg ctcatattct 3780
tttcagagca ctaaggagag aacattatgt cgactcaggg aggagaaaaa caactcacca 3840
agccttgttt ttcttttcct ctgagtttgc cttaccagct ggagaaaagt gatcccaacc 3900
tcttttcaac ttctccaacc cgaaccaggt gtgattgtga gtccacccctt tgccattagg 3960
atgccagcac tcagtaaccc gctttgttag tttgcttttt tggacaaccc actaccagat 4020
cggcagtgca tttccctcac tacactcaca catgcactct gcataaaagc taataataag 4080
gtcatcctga tttttgtttt ttcttttttg ggaaaacatc actttgatac tatgtatggt 4140
tttctttggt cttaagtggt catcacttga atcctatgac ctactaatta gttaacactg 4200
cttaaaggaa tgaaaagtat ttgaaattaa catgggtgtg aatctaccct aaaatgaggg 4260
ccacctctcc aaacaaattc cagaaaaccc acctcttcaa aaaagtacca ccaaaaagaa 4320
atataaatcc ttagatggat agaaattcct caagagaaca gtcacttaaa catttagtag 4380
tttcataatg ttgaatttgt atagtacatg catagtatgt gcaaagccta ttttgaccat 4440
atttctctct aacctttttca cccttcttgg tcaactgaaa tgaattcaat attactcatt 4500
ttgtttgctt cattctttag acaattttcc aaagcataca aaccttacaa accttcctca 4560
atttcaaaat aatgtgacta ttttagcaat attttcaggt tgacacatca aagtatttta 4620
gaaaattaaa acttagggct gccactctct atactgcttt accaataact taaaaacaaa 4680
caaagaagga ccaggggctt ggacatataa gctatcttcc catcagtctc agcttaacta 4740
agtatacatt atttagtcat gtaatgtgtt ctgtgggtga attactccct catcccaata 4800
tttataaatt cactcattta gctaagtgtt tatgcctggc cttaaataat ttagtacact 4860
tgaaccctct tataaccctg ctcctccctg cattaacttg aatacttcta aggtaagact 4920
gaaccccacc atgactctac acagaaattg ttcctaaaag ataccagcgt tagaaggagt 4980
tgaattttat ttattggata catacatata tgtataatat ataatacaca tatgtgtatt 5040
atacattatc atacatatat gtattatata ttacacatat atgtataata tataatacac 5100
atatgtatta tatataatac atatatgtat aatatatgtg tttcatatgt atgtatttgt 5160
ttaattttgt atacagatta ggagaagcag tttttgtttt gtttttcctt taggaaatca 5220
tattccctaa ttggaatggg aaagaggaaa gaaccataag ctggagctta cttccttttc 5280
taccgacaag gaacccaaac ttcaaaactt atttgtcaac ataaaaaaga caataataaa 5340
aacaacaact ttagaacgtt caggacaaag ccttcaaagc cttcaatgcc ctgaagcagg 5400
ttttagaatg gctgtcctct caaattgctt tttcaagtgt actgacccgc actttgtctt 5460
cagtcacacc taagttttcg gcaagttccc ttctgtggag agaagatcac acatggttag 5520
tattcaaagt tgtggatgaa atgaaatata tagtatgtac tatttacttc atgcttgttt 5580
tacaatttat aatctcccct cacacctccc ccaagtatat acttttctct aattcccagc 5640
tccatggttg ctttagaaat ggtttaccct catcacgaaa tttaaggtga cgttaacaac 5700
tcagtaatca agagaaatac cttttttttt ttaaattgag acaaggtctc actctgtctc 5760
ctaggctgga gtgcagtggt gtgatttcag ctcactgcaa cctccgcctc cggggttcag 5820
acgattctcg tgcctcagcc tcccgagtag ctgcgattac aggcacatac caccatgccc 5880
agttgatttt tgtattttta gtagagatgg ggttttgcca tgttggccag gctggtctcg 5940
aactcctgcc cgtctcagcc tcccaaagtg ctgggatttg gggcatgaac caccgcaccc 6000
ggccaagatg aataatttaa tgcattatta ttattttat tattattatt tgagacaggg 6060
tctcactgtc gtctatgttg gagtgcagtg gcaggatcac tgctcactgc agcctgcatg 6120
tcctgggctc gaacgatcct cctgcctcag ccttccaagt ggctgggagt acaggcacac 6180
accaccacac ccacatggct aattttttaa gttttattta gagacggggt tttgccatgt 6240
tgcccaggct gttcttgaac tcctggactc aagcaacctt cccaccttgg cctcccaaaa 6300
gcgctggaat tacaggcctg agccaccgtg cctggcccta atgcactatt ttaataaata 6360
acaattaatg caaaaatctg tgatgaggac caggcactgt ggctcaggcc tgtaatccca 6420

```
gcagtttggg aggccgaggc aggcaaattg cttgagccca ggagtttgag actagcctgg 6480
gcaacacggc gaaaccttat ctctacacac aaaaaaaata caaaaattag ccaggtgtgg 6540
tggcctgtgc ctgcagtccc agctactcag gggctgaca cgggaggatg gcttgaaccc 6600
aggaagcaaa tgttgcagag agctgaaatc gcactgctgc actccaacct gggccacaga 6660
gagagactct gtctcaagac aaaacaaaaa aaccagaaaa acaaaaaacc aaccaaacaa 6720
acaaaaaaaa actatgatga acaaattatc aaaattttaa ataaaggaag gatctagcac 6780
tgtagttgca tgacagtacc tcattctcct taccccaatt tcaataaaat tttatttata 6840
aaaacagacc acagctgggt gtggtggctc actcctataa tcccagcaac tcaggaggct 6900
gagatgggag gattgcttgg gtgacagatc cccactcaa caaaaacaac aacaacaaca 6960
aaaacaggcc atcatcacag gtaataaaag aaaaaataca taacttggac tatatcaaaa 7020
tttaaaactt ctgtatatca aaagatgcaa tgaacagagt aaaaagacaa ctcatagaat 7080
ggaaggaaat atttgcaaat cacatctgat aaggggttaa tatccagagt gtataaagaa 7140
ctcctacaac ccaataacca aaaaaaaaga aagaaagaaa gaaaaagcca ctcagatttt 7200
aaaatgggta aaggacttaa agagatattt ctccaaagaa gatatacaag tggccactaa 7260
gcacatgaaa ggatgcacaa catcactaat cattagggaa aagcaaatcg aaactacaat 7320
gaagtatcac ctcacaccca ttaggatggc tatgtaaaaa accccagaaa ataacaagtg 7380
ttggtgagga tgtggagaaa ctggaacccc catgtactgt tggtgtgcac ctgtatctat 7440
aaaatggaat attatttagc cttaaaaagg aaggaaattc taatatatgc tgcgatatgg 7500
atgaaccttg aagaccttat gctaagtgaa ataagtcagt gacaaaaatg caaatactgt 7560
atgattctac ttacatgaga tacctagagt agtcaaaatc atagagacat aaaatagtag 7620
aatggtggtt gccaagggct ggggaaaggg ggaaaagggg agttgcttaa ctggtataga 7680
gacttagctt ggcaagatga gaagaattct agagatctat tgcacaacaa tgtgaacata 7740
cttaacacaa ctgaactcta tacttaaaaa gtggtttgga cggtaaattt catatttccg 7800
tgtattttac cacatcttta taaaagggag gcacggacta gtttccaggt ttcattcaca 7860
taaacattgc aataaaacat ttaccttgat gcccaggagg taaatatccc cctccacacc 7920
agcacaaagg caggcaagga cccccagtgg ctttttcctc atgattgggt ggggcaaggg 7980
agagaaaaag atgcctcgaa acgaacttgg agatctcgtg gctcctggag caggccactt 8040
accttgtggg cacatcaggg tattgagtgt gtcggaaaac actttccagc tcctccacct 8100
gcaacagcgt gaacttcgtg cgccgagttc gtggctgcat gttctcgggc tgcggaccct 8160
ccatggccgc ctgggccggc tcctccggcg gggctgcgg ctgctgccga ggctcctggt 8220
ttccaccgcc gccctcgggg atcatgccgc catcgcggtt catgccgttc tcgtggttca 8280
caccgccctc aggttcata ttacccatga ggcctggagc tccttggcca acatggcctt 8340
ctgcgcttga tgctgccccc agctgaggtg tggggcttat ttttacctgg tatacactca 8400
ggcagtagaa cacggtgtcg tggacgagcg aacgcgccat ggctggagcg ctgcgcccct 8460
gcacaaactc cgtggcgtct gcagctggag tgggggttag agggtggagc tagttcctgt 8520
tctcatgctt ggtattggtt acagttgcaa tgagtgggac ttgcttatgc gcacaagcaa 8580
gagagggaat ggagaggagt ggggggatgg gaagttgggg ggtgcgggtg gggagtgggg 8640
gtgttgcagg tgggagtggg gggttgtgag tgtggggtgg ggtgcaggtg gggatggggg 8700
tgtgggtgga gggtgggggg tgcacagtga gggtgggggt tgcgggtgag ggtaggggtt 8760
gtgggttggg gtgggggttg ccggtggggg tacatggtgg gggtgggggt agcgggtgga 8820
gatgggaggt gtgggtggag ggtgcgtggt gggggtaggg gttgtgggtg ggggtgaggg 8880
gtgtggtatg ggtcgtgggt gggggtggca gttgagggtg gagtggggtg gccaaaacac 8940
aggggcagtg tggagaagaa aagggccaat aggaggcata tatgtatgca acatggggcc 9000
ccagcttgca gctttgctga ctacacccta ctcgggccta gttattaccc tgaggaaagc 9060
tgatttgggg gctcagaggg gaggtgagat ctcacggtga ccataggacg ccttgagtaa 9120
aagtttggag aatatctcat ggcctgaccc tccatatttg gcagcatgca cagggcgcgg 9180
gctattaatt aagcagaaat gattgactgg gggctgcttg ttcagagttc cagcaaaggc 9240
actgaaagca gagctgccat gctctcttca gtgctgggat cgggatcttg gagatgggca 9300
tgcagagcat tctgggtggt aagatgtgct ctgcaagaaa tctaacgcac cctttgagaa 9360
agtcaacaca gaataaacac gaggctgaat ctgttagcct gagactgaat atctttggct 9420
atgcaagaga aacctgtact catggcaaaa tggagtgcta taaggacaag caaaaaataa 9480
ataaataaat aaaatcgggg atggtatagg aagagcacca gtaagggcat acctgccaaa 9540
aatctccaat cttgggatgg agatttggga tttatggata tgcagcttac tggatgtggg 9600
gccacttctg ctccacagag ccttgtaact acacagcctt cctaccactg accccaataa 9660
gcccaattac gaagaaaaac cctgaagagc ctggtgcagt ggctcctgca ctagtcccag 9720
ctactcagga ggctgagatg ggaggatcac ttgaacccag gagtttgagg ctgtggtgag 9780
ctagaatcac atggcagcac tccagcctgg gcaacagaca gaggcccctt ttctttaaaa 9840
taaataataa aataagaaat aaaatgaaaa tgaaagaaag gaaagcgcta agagagtctg 9900
tcatgaggaa gggcatggag atgtcttttg agggtggaca actcatgaat ccttaatttt 9960
tctagagatt gtgtgtgtgc tcttaagtga tgttatatac tttattttgt tttttaaaaa 10020
tatttttaaa aattttattt ttaaatgttc ttttaaaaac tttctgtatc tatttatatc 10080
tattggttat ttgaggattt tttggcagca tatataaata tgcagaccct ttgagtctgt 10140
agcctaccaa gagagatagc tctcgtcttc atggtgattc tgagcatgga aaggcccttg 10200
```

```
cacttggcag catgacaagg actaagccac tcgctccatt aattgactgc catccactgg 10260
gctaagtgag atccttgcgt tctatcccta gtgagagaag agagaggaag aagaagaaaa 10320
atagaaagat aataagaaaa tagaaaaaga aatgaataaa tgtacattgt ggggagcagg 10380
aaaggactac cagtaatggg aggcatcagc taggagcaca gatccgaagc atgactcact 10440
gtgtgtccta ggacactgga tgaatctatc tggttctcag cttcctcacc tataaaatgg 10500
agataacaac agtgtctcga tcatagggtt ttcatgagag ttcaatgagg caaggcatac 10560
atgtaactga acacagctcc gactgctcac cagttgcaaa gtccagtgaa caagaacgac 10620
gtctggtaga aagaaagtgg ctttattcca gagctagttg aggggaagta gtacaggctg 10680
ccttgaggaa gccactaaag cctttggggc agaaggcagg agctttgaaa gtggggcttg 10740
gcgtgaatgg catgcagggg agagggcgat gaagtgcaga gtctatgtga cttgcttcgg 10800
atgtcttatc tatcaggtgg tctggctggc accgtcacgg gcagagctag gttgtaagtt 10860
gaggcaatct caatttgcct cctggtagga gagagttctg gaggttcctg gtttgcttta 10920
aggttcggtc tctgtaactt ctaagtaaac atgtagttag ataagctt             10968
```